# EUROPÄISCHE PATENTSCHRIFT

(11) **EP 0 658 164 B1**
(45) Veröffentlichungstag und Bekanntmachung des Hinweises auf die Patenterteilung: **04.04.2001**
(21) Anmeldenummer: 94922869.6
(22) Anmeldetag: 24.06.1994
(51) Int. Cl.: C07H 1/08, C12N 15/10, C07H 21/00

(54) **VERFAHREN ZUR CHROMATOGRAPHISCHEN REINIGUNG UND TRENNUNG VON NUCLEINSÄUREGEMISCHEN**
CHROMATOGRAPHIC PURIFICATION AND SEPARATION PROCESS FOR MIXTURES OF NUCLEIC ACIDS
PROCEDE DE PURIFICATION ET DE SEPARATION CHROMATOGRAPHIQUES DE MELANGES D'ACIDES NUCLEIQUES

(30) Priorität: 01.07.1993 DE 4321904
(43) Veröffentlichungstag der Anmeldung: 21.06.1995
(73) Patentinhaber: QIAGEN GmbH, 40724 Hilden (DE)
(72) Erfinder: COLPAN, Metin, D-45219 Essen (DE); SCHORR, Joachim, 40721 Hilden (DE); HERMANN, Ralf, D-50674 Köln (DE); FEUSER-FRANK Petra, 46509 Xanten (DE)
(74) Vertreter: Meyers, Hans-Wilhelm, Dr.Dipl.-Chem.
(86) Internationale Anmeldenummer: EP9402056
(87) Internationale Veröffentlichungsnummer: WO9501359

(56) Entgegenhaltungen:
- EP-A- 0 389 063
- WO-A-93/11221

## Beschreibung

Gegenstand der vorliegenden Erfindung ist ein Verfahren zur chromatographischen Reinigung und Trennung von Nucleinsäuregemischen gemäß Oberbegriff des Anspruchs 1, die Verwendung des Verfahrens zur Reinigung von Nucleinsäurefragmenten, die Modifizierungsreaktionen unterzogen worden sind, eine Vorrichtung zur Durchführung des Verfahrens, eine wäßrige Lösung, die im erfindungsgemäßen Verfahren einsetzbar ist sowie die Verwendung dieser Lösung.

Die Adsorption von Nucleinsäuren an Glas- oder Silicagelpartikeln in Gegenwart von chaotropen Salzen ist bekannt (Vogelstein, B. and Gillespie, D. (1979) Preparative and analytical purification of DNA from agarose., Proc. Natl. Acad. Sci. USA 76: 615 - 619). Gemäß dieser Methode wird DNA aus Agarosegelen sowie RNA- und DNA-Präparate aus verschiedenen Extrakten unter Verwendung von hohen Konzentrationen chaotroper Salze in Natriumiodid, Natriumperchlorat oder Guanidinthiocyanat isoliert und gereinigt (Boom, R. et al. (1990) Rapid and simple method for purification of nucleic acids, J. Clin. Microbiol. 28, 495 - 503 und Yamado, O. et al. (1990) A new method for extracting DNA or RNA for polymerase chain reaction. J. Viro. Methods 27, 203 - 210). Zwar sind die genauen pysikalischen Vorgänge, die zu einer Adsorption der Nucleinsäuren in Gegenwart chaotroper Reagenzien an mineralische Träger führen nicht im Detail geklärt, jedoch geht man davon aus, daß die Ursache für die Adsorption in der Störung übergeordneter Strukturen des wäßrigen Milieus zu suchen ist. Dabei wird die in Lösung befindliche Nucleinsäure an der Oberfläche der Glas- bzw. Silicalgelpartikel adsorbiert oder denaturiert. Die Adsorption ist in Anwesenheit von hohen Konzentrationen chaotroper Salze fast quantitativ. Die Elution der adsorbierten Nucleinsäuren erfolgt in Anwesenheit von Puffern mit geringer Ionenstärke (Salzkonzentration). Durch die Verfahren des Standes der Technik werden Nucleinsäuren und Fragmente einer Größe von 100 Basenpaaren (bp) bis 50.000 Basenpaaren (bp) ermöglicht. Es war bislang jedoch nicht möglich, kurze einzel- oder doppelsträngige Nucleinsäurefragmente (100 bp und kleiner) von sehr kurzen (20 bis 40 Nucleotide) einzelsträngigen Oligonucleotiden (Primern) quantitativ zu trennen.

Diese Nucleinsäuregemische entstehen typischerweise als Amplifikationsprodukte zum Beispiel gemäß der Polymerasekettenreaktion (PCR). Die aus dieser Reaktion entstehenden Produkte werden häufig anschließend molekularbiologisch analysiert, indem die üblichen Techniken wie DNA-Sequenzierung, DNA-Hybridisierung, Klonierung, Restriktion und Transformation durchgeführt werden. Damit lassen sich analytische Parameter wie Aussagen über Genmutationen für die genetische Beratung oder Erregernachweise in der medizinischen Diagnostik (HIV) gewinnen. Um das Potential dieser Diagnostikverfahren nutzen zu können, ist eine quantitative Trennung bzw. Reinigung dieser häufig recht kleinen DNA-Fragmente (100 Basenpaare) sehr wichtig.

Die zur Zeit zur Verfügung stehenden Reinigungsmethoden basieren auf der Ultrafiltration, der High Pressure Liquid Chromatography (HPLC) oder der Extraktion von Nucleinsäurefragmenten aus Agarosegelen in Anwesenheit chaotroper Salze durch Niederschlagen auf Glas- oder Silicagelpartikel. Diese Methoden sind jedoch nur mit niedriger Effizienz zur Trennung von Nucleinsäuregemischen bestehend beispielsweise aus einem kurzen doppelsträngigen DNA-Fragment (100 bp) und einem kleineren einzelsträngigen Oligonucleotid (zum Beispiel 39 mer) geeignet.

Das technische Problem der vorliegenden Erfindung besteht darin, ein Verfahren bereitzustellen, mit dem es möglich ist, die oben genannten Nachteile des Standes der Technik zu vermeiden. Gelöst wird das Problem durch ein Verfahren zur chromatographischen Reinigung und Trennung von Nucleinsäuregemischen wobei das Nukleinsäuregemisch
- aus einer wässrigen Adsorptionslösung mit hoher Salzkonzentration (Ionenstärke) und mit 1 bis 50 Vol.-% aliphatischen Alkohols einer Kettenlänge von C₁-C₅ und/oder Polyethylenglykol (PEG) und/oder hydrophober, anorganischen und/oder organischen Polymeren und/oder organischer Säure, wie Trichloressigsäure (TCA),
- an einem porösen oder nicht porösen mineralischen Träger aus Metalloxiden und/oder Metallmischoxiden, Silicalgel, Materialien, die überwiegend aus Glas bestehen, Aluminiumoxid, Zeolithe, Titandioxid, Zirkondioxid - ohne vorherige Reinigungsschritte - adsorbiert wird,
- gegebenenfalls mit einer Waschlösung gewaschen und danach
- mit einer Lösung geringerer Salzkonzentration (Ionenstärke) eluiert wird und die erhaltene Nucleinsäuren oder Nucleinsäurefraktionen gesammelt werden.

In einer bevorzugten Ausführungsform werden die Ionenstärken der Salzlösungen mit 1 bis 10 M Lithiumchlorid, Natriumchlorid, Kaliumchlorid, Natriumacetat, Reagenzien wie z.B. Harnstoff und/oder Mischungen davon eingestellt.

Bei weiteren bevorzugten Ausführungsformen beträgt die Partikelgröße des mineralischen Trägermaterials 0,1 µm bis 1.000 µm und/oder weisen verwendete poröse mineralische Trägermaterialien eine Porengröße von 2 bis 1.000 nm auf und/oder liegen die porösen oder nicht porösen Trägermaterialien, insbesondere Zeolithen, in Form von losen Schüttungen vor, oder sind die porösen oder nicht porösen Trägermaterialien, insbesondere Zeolithen, als Filterschichten ausgebildet in Form von Filterschichten aus Glas, Quarz oder Keramik und/oder einer Membran, in der Silicagel angeordnet ist und/oder liegen als Partikel oder Fasern aus mineralischen Trägern und Geweben aus Quarz oder Glaswolle vor.

Bei den obigen Verfahren stammen die zu trennenden und zu reinigenden Nucleinsäuren bevorzugt aus biologischen Quellen wie Zellkulturen, Geweben jeder Art, Körperflüssigkeiten wie Blut, Plasma, Serum, Urin, Faeces, Mikroorganismen wie Bakterien, insbesondere Mycobakteriumtuberkulosis, Viren wie Cytomegalie-Virus, HIV, Hepatitis B, Hepatitis C, Hepatitis-δ-Virus, wobei die Nucleinsäuren durch Polymerasekettenreaktion (PCR) erhaltene Produkte, Plasmid-DNA, genomische DNA, RNA, Nucleinsäuren aus Mikroorganismen wie Plasmid-DNA, chromosomale DNA oder RNA und/oder Nucleinsäuren aus Sequenzanalysen sind.

Insbesondere sind auch Verfahren eingeschlossen, bei denen die Nucleinsäuren nach Fraktionierung zu weniger als 10% kürzer als 10 kb sind, oder bei denen die Nucleinsäuren Oligonucleotide sind und/oder bei denen nach Lyse einer nukleinsäurehaltigen Probe als Quelle der zu reinigenden und isolierenden Nucleinsäure die Einstellung von Adsorptionsbedingungen an Silicagel in einem einzigen Schritt erfolgt, wobei die Adsorptionsbedingungen durch Lyse der Proben In dem Puffersystem, das zur Adsorption eingesetzt wird, eingestellt werden.

Das Verfahren erlaubt in einer besonderen Ausführungsform auch die Reinigung und Trennung von Nucleinsäurefragmenten nach Modifizierungsreaktionen.

Das erfindungsgemäße Verfahren nutzt die an sich bekannte Eigenschaft von Nucleinsäuren aus, in Gegenwart chaotroper Salze, Salzlösungen hoher Ionenstärke (hoher Konzentrationen), Reagenzien wie z. B. Harnstoff oder von Mischungen dieser Substanzen auf mineralischen Träger niederzuschlagen und durch Einwirkung von Lösungen geringer Ionenstärke (Salzkonzentration) zu eluieren. So schlägt die (PCT/EP 92/02775) WO-93/11221 der Anmelderin vor, ein Nucleinsäuregemisch zunächst in einem Medium niederer Ionenstärke auf einem Anionenaustauschermaterial zu adsorbieren, danach die Nucleinsäure mit einem Puffer höherer Ionenstärke zu desorbieren und danach im Puffer mit dieser höheren Ionenstärke in Gegenwart von niederen Alkoholen und/oder Polyethylenglycol und/oder organischer Säuren, wie Trichloressigsäure (TCA), die Nucleinsäuren auf einem mineralischen Trägermaterial zu adsorbieren. Die Nucleinsäuren werden dann vorzugsweise mit Wasser oder einer Pufferlösung geringer Ionenstärke eluiert.

Es hat sich nun herausgestellt, daß zur Trennung von Nucleinsäuren die vorherige Reinigung an Anionenaustauschermaterialien unterbleiben kann. Überraschenderweise läßt sich auch durch die Adsorption von Nucleinsäuren in Gegenwart chaotroper Salze hoher Konzentration und Desorption der Nucleinsäuren mit Lösungen geringer Ionenstärke eine hervorragende Fraktionierung eines Nucleinsäuregemisches erzielen.

Durch das erfindungsgemäße Verfahren wird es mithin ermöglicht, in einem Arbeitsschritt durch Adsorption der zu trennenden Nucleinsäuren und Elution in effizienter Weise interessierende Nucleinsäurefraktionen zu erhalten, ohne vorherige Reinigungsschritte.

Sollen nucleinsäurehaltige Proben als Quellen der zu reinigenden und isolierenden Nucleinsäuren dienen, werden diese Quellen in an sich bekannter Weise aufgeschlossen, beispielsweise durch Detergenzbehandlung oder mechanische Einflüsse wie Ultraschall oder Zerkleinern. Dabei kann die zur Aufnahme der Nucleinsäuren verwendete Lösung bereits chaotrope Salze in hoher Konzentration enthalten. Nach Entfernung eventuell vorhandener grober Zellbestandteile durch Zentrifugation oder Filtration (WO 93/11218 und WO 93/11211) wird die Lösung dann mit einem mineralischen Trägermaterial in Kontakt gebracht, um aus der Lösung mit hoher Ionenstärke chaotroper Salze die Nucleinsäuren auf dem mineralischen Träger zu adsorbieren.

Eine Abwandlung des erfindungsgemäßen Verfahrens besteht darin, den Aufschluß der Nucleinsäuren direkt in dem Puffersystem, das zur Adsorption eingesetzt wird, durchzuführen. Dann wird eine besonders günstige Nucleinsäureverteilung gewinnbar.

Üblicherweise werden Nucleinsäuren aus eukaryontischen und/oder prokaryontischen Zellen (darunter auch Protozoen und Pilze) und/oder aus Viren gewonnen. Dabei werden die Zellen und/oder Viren beispielsweise unter stark denaturierenden und gegebenenfalls reduzierenden Bedingungen aufgeschlossen (Maniatis, T., Fritsch, E.F. & Sambrook, S., 1982, Molecular Clonin Laboratory Manual, Cold Spring Harbor University Press, Cold Spring Harbor).

Eine besondere Ausführungsform der Erfindung eignet sich insbesondere zur Plasmid- oder Cosmid-DNA-Präparation aus E.coli. Nach der Lyse der E.coli-Zellen mit Natronlauge/SDS wird mit 0,2 - 0,9 M Kaliumacetat (KAc) neutralisiert.

Üblicherweise wird nach der Lyse der Zellaufschluß mit SDS mit 3 M Kaliumacetat neutralisiert. Um die Zellbruchstücke abzuzentrifugieren, wird dem Zell-Lysat dann 5 M Guanidinhydrochlorid oder eine andere chaotrope Hochsalzlösung zugegeben. Bei Minipräparationen von E.coli ergibt dies ca. 2 - 3 ml der an Silicagel zu adsorbierenden Probe, was jedoch nachteilig ist, da es dann in mehreren Stunden abzentrifugiert werden muß.

Das erfindungsgemäße Verfahren verwendet nach der Natronlauge/SDS Lyse z. B. Salzlösungen mit vorzugsweise
0,2 M KAc / 5,5 M GuHCl,
0,2 M KAc / 5,5 GITC,
0,2 M NaAc / 6 M Na ClO₄,
0,2 M NaAc / 6 M GuHCl sowie
0,2 M NH₄Ac / 6 M NaClO₄.

Dadurch wird eine Neutralisation der Zell-Lyse und gleichzeitige Einstellung der Probe auf Hochsalzbedingungen in Silicagel erreicht, wodurch eine wesentliche Arbeitserleichterung in der täglichen Praxis erfolgt. Weiter wurde überraschenderweise festgestellt, daß die Adsorption von Nukleinsäuren an Silicagel auch in Anwesenheit von anionischen oder kationischen oder neutralen Detergentien, wie z. B. SDS, NP40, Tween 20 Triton X-100, CTAB in Kombination mit chaotropen Salzen stattfindet, oder durch die Anwesenheit dieser Detergentien die DNA-Ausbeute sogar noch erhöht.

Weit verbreitet ist der Aufschluß der Zellen mit Detergenzien als denaturierende Reagenzien und die Verwendung von bestimmten Enzymen zum Abbau der Proteinstrukturen und nucleinsäurespaltenden Enzymen. So werden beispielsweise Natriumdodecylsulfat (SDS) und EDTA als denaturierende Agenzien verwendet und Proteinase K zum Abbau von Proteinen. Das Ergebnis dieses Aufschlußverfahrens ist meistens eine hochviskose gallertartige Struktur, aus der die Nucleinsäuren mittels Phenolextraktion isoliert werden. Die Nucleinsäuren bleiben dabei in großer Länge erhalten und werden nach Dialyse und Präzipitation aus der wäßrigen Phase entfernt. Dieses Aufschlußverfahren ist gegenüber Nicht-Nucleinsäure-Strukturen so aggresiv, daß dem Verfahren auch Gewebestücke unterworfen werden können.

Wegen der arbeitsintensiven Technik mit mehrfachem Wechsel der Reaktionsgefäße ist diese Methode jedoch für große Probenaufkommen und Routinepräparationen ungünstig. Dieses Verfahren ist zwar automatisierbar, jedoch bewältigt eine handelsübliche Apparatur gegenwärtig etwa 8 Proben gleichzeitig in vier Stunden (Applied Biosystems A 371). Das Verfahren ist somit teuer und eignet sich nicht für die Durchschleusung großer Probenserien. Weiterhin ist nachteilig, daß die Folgereaktionen, wie enzymatische Amplifikation, infolge der großen Längen der isolierten Nucleinsäuren gestört sind. Darüber hinaus sind die anfallenden Lösungen hochviskos und schwer handhabbar. Insbesondere DNA sehr großer Länge ist eher störend, da die mit dem Verfahren gemäß dem Stand der Technik gewonnenen Nucleinsäuren gesondert zerkleinert werden müssen, um weiterverarbeitet werden zu können.

Der Aufschluß von eukaryontischen und/oder prokaryontischen Zellen und/oder Viren in alkalischem Milieu in Gegenwart von Detergenzien, ist technisch zwar einfach, liefert jedoch auch Nucleinsäuren mit großer Länge, die wie oben geschildert von Nachteil sind.

An die Rohpräparation der Nucleinsäuren schließen sich Folgereaktionen an. Diese Folgereaktionen erfordern eine bestimmte Qualität der Nucleinsäuren. So müssen diese weitestgehend unversehrt sein, die Ausbeute der Präparation muß hoch und reproduzierbar sein, außerdem müssen die Nucleinsäuren in hoher Reinheit, frei von Proteinen und Zellmetaboliten vorliegen. Der Präparationsweg muß einfach und wirtschaftlich sein und die Möglichkeit zur Automation bieten. Die Präparation der Nucleinsäuren muß ohne die Gefahr von Kreuzkontamination anderer Proben möglich sein, insbesondere wenn enzymatische Amplifikationsreaktionen, wie Polymerase Chain Reaction (PCR) (Saiki, r., Gelfand, D.H., Stoffel, S., Scharf, s.J., Higuchi, R., Horn, G.T., Mullis, K.B. & Ehrlich, H.A. (1988), Science 239, 487 - 491) und Ligase Chain Reaction (LCR) ( EP-A-8 83 11 741.8), Anwendung finden. Für diese Folgereaktionen ist es wünschenswert, die Nucleinsäuren in nicht zu großer Kettenlänge zu erhalten, die Zellen möglichst quantitativ aufzuschließen und im übrigen die oben genannten Nachteile der im Stand der Technik bekannten Aufschlußverfahren zu vermeiden.

Es ist mithin wünschenswert, daß ein Verfahren die Isolierung und Konzentrierung von Nucleinsäuren aus intakten eukaryontischen und/oder prokaryontischen Zellen und/oder Viren oder aus Körperflüssigkeiten ermöglicht. Insbesondere soll die dabei anfallende Nucleinsäure sich durch nicht zu große Kettenlängen auszeichnen, in wenigen Schritten isolierbar sein und direkt den erforderlichen Folgereaktionen unterworfen werden können.

Die weiter oben angegebene Modifikation des erfindungsgemäßen Verfahrens die dies ermöglicht, besteht darin, daß die Quellen der Nucleinsäuren wie eukaryontische und/oder prokaryontische Zellen und/oder Viren lysiert werden.

Der Aufschluß nucleinsäurehaltiger Quellen, wie eukaryontische und/oder prokaryontische Zellen und/oder Viren, kann dabei vorzugsweise durch physikalische oder chemische Einwirkung erfolgen. Dabei kann die Lyse entweder mechanisch wie mit Ultraschall oder durch osmotischen Schock oder chemisch mittels Detergenzien und/oder chaotropen Agenzien und/oder organischen Lösungsmitteln (z.B. Phenol, Chloroform, Ether) oder alkalischem Aufschluß erreicht werden.

Diese Verfahrensweise führt zur Präparation von Nucleinsäuren mit hoher Reinheit und erlaubt es, eine qualitativ und quantitativ reproduzierbare Analytik durchzuführen, insbesondere in Kombination mit enzymatischen Verfahren zur Amplifikation von Nucleinsäuren. Es hat sich gezeigt, daß Aufschlußmethoden mit Detergenzien und/oder chaotropen Agenzien, konzentrierten Salzlösungen, Reagenzien wie Harnstoff, Mischungen dieser Substanzen und/oder organischen Lösungsmitteln oder physikalische Aufschlußmethoden, wie Erhitzen einer Probe, die Folgeanwendungen erleichtert. Man erhält bei Anwendung des erfindungsgemäßen Verfahrens zum Beispiel kürzere zelluläre DNA (< 50 kb) beziehungsweise Gesamtnucleinsäuren aus Zellen und/oder Viren und/oder Körperflüssigkeiten. Die Reinigungsmethode (d.h. die Bedingungen der Bindung und Elution der Nucleinsäuren) führt zu einer Fragmentierung der Nucleinsäuren.

Die Kombination aus chaotropen Agenzien hoher Ionenstärke und hydrophoben organischen oder anorganischen Polymeren und/oder Alkoholen und/oder Trichloressigsäure (TCA) im Adsorptionspuffer gewährleistet, daß die Nucleinsäuren im Gegensatz zu herkömmlichen Reinigungsverfahren nach der Lyse quantitativ und hochspezifisch an der Oberfläche des mineralischen Trägermaterials wie Quarzfasern fixiert werden und so vor weiterführenden Angriffen der Nucleasen geschützt sind, während kontaminierende Bestandteile des Lysates nicht binden. In diesem fixierten Zustand der Nucleinsäuren können restliche kontaminierende Bestandteile leicht ausgewaschen werden, gefolgt von der Elution der sauberen Nucleinsäure in einem kleinen Volumen. Man erhält so reproduzierbare Kettenlängen von durchschnittlich 20 bis 40 kb. Weniger als 10% sind kürzer als 10 kb unter den Aufschlußbedingungen, wie sie in den Beispielen 7 bis 9 beschrieben werden. Dies stellt eine optimale Längenverteilung für eine anschließende enzymatische Nucleinsäureamplifikation dar.

Die spezielle Kombination von Salzen, insbesondere chaotropen Agenzien, und Alkoholen ermöglicht es erstmals, Nucleinsäuren eines breiten Kettenlängenspektrums (10 - 100.000 Basenpaare) gleichzeitig zu isolieren und zu reinigen.

Die wäßrige Adsorptionslösung hoher Salzkonzentration enthält 1 bis 50 Vol.-% aliphatischen Alkohols einer Kettenlänge von 1 bis 5 C-Atomen oder Polyethylenglykol.

Als mineralische Träger kommen poröse oder nicht poröse Materialien auf Basis von Metalloxiden und Metallmischoxiden in Frage, wie solche aus Silicagel, Materialien, die überwiegend aus Glas bestehen, Aluminiumoxid, Zeolithe, Titandioxid, Zirkondioxid. Insbesondere Zeolithe haben sich als mineralisches Trägermaterial bewährt.

Gegebenenfalls kann das mineralische Trägermaterial mit den daran adsorbierten Nucleinsäuren mit einer Lösung gewaschen werden, die aufgrund eines relativ hohen Alkoholgehaltes eine Desorption der Nucleinsäuren verhindert.

Danach werden die adsorbierten Nucleinsäuren mit einem Puffer geringer Salzkonzentration (Ionenstärke) eluiert und die erhaltenen Nucleinsäuren oder Nucleinsäurefraktionen gesammelt.

Als chaotrope Salze kommen Natriumperchlorat, Guanidinhydrochlorid (GuHCl), Guanidinisothiocyanat (GTC), Kaliumiodid in Konzentrationen von 1 bis 8 M in Betracht. Nutzbar sind ebenfalls konzentrierte Salzlösungen > 1 M NaCl, KCl, LiCl etc., Reagenzien wie beispielsweise Harnstoff (> 1 M) und Kombinationen dieser Bestandteile. Die in der Lösung der chaotropen Salze vorhandenen niederen Alkohole sind Methanol, Ethanol, Isopropanol, Butanol und Pentanol in Mengen von 1 bis 50 %, insofern sie in diesen Bereichen mit Wasser mischbar sind. Die vorzugsweise verwendbaren Ethylenglykole weisen Molekulargewichte von 1.000 bis 100.000, insbesondere von 6.000 bis 8.000 auf. Das Polyethylenglykol kann dem Puffer hoher Ionenstärke in Mengen von 1 bis 30 % zugesetzt sein.

Die Partikelgröße der mineralischen Trägermaterialien beträgt vorzugsweise 0,1 µm bis 1.000 µm. Werden poröse mineralische Träger verwendet wie beispielsweise poröses Silicagel, poröses Glas, poröses Aluminiumoxid, Zeolithe weisen die Poren vorzugsweise eine Porengröße von 2 bis 1.000 nm auf. Das Trägermaterial kann beispielsweise in Form loser Schüttungen vorliegen und mit den die zu trennenden und reinigenden Nucleinsäuren enthaltenden Lösungen in Kontakt gebracht werden.

Vorzugsweise sind jedoch die porösen und nicht porösen Trägermaterialien als Filterschichten ausgebildet und in einem Hohlkörper mit Ein- und Auslaßöffnung angeordnet. Die Filterschichten bestehen entweder aus gerichteten (gewebten) oder ungerichteten Fasern aus Glas, Quarz, Keramik oder anderen Materialien wie Mineralien oder aus einer Membran, in der Silicagel angeordnet ist.

Das erfindungsgemäße Verfahren ist in hervorragender Weise geeignet, Nucleinsäuregemische zu trennen, insbesondere auch kurzkettige Nucleinsäuren, die sich in den Kettenlängen nur geringfügig unterscheiden. So lassen sich beispielsweise DNA-Fragmente einer Größe von beispielsweise 100 bp von kleineren einzelsträngigen Oligonucleotiden zum Beispiel einem 39 mer trennen. Dabei wird die DNA-Ausbeute dann um 60 bis 70 % gesteigert, verglichen mit anderen konventionellen Reinigungsmethoden, wie Ultrafiltration, HPLC oder Verwendung chaotroper Salze allein.

Bei Anwendung des erfindungsgemäßen Verfahrens, bei dem der Aufschluß der Nucleinsäuren enthaltenden Quellen im Aufnahme-(Adsorptions-)puffer erfolgt, wird eine Nucleinsäurepräparation mit bestimmten Längenspektrum der Nucleinsäure möglich.

Das erfindungsgemäße Verfahren erlaubt es, Nucleinsäuregemische jeglicher Herkunft zu bearbeiten. So lassen sich Nucleinsäuren aus biologischen Quellen wie Geweben jeder Art, Körperflüssigkeiten wie Blut, Faeces nach entsprechender Probenvorbereitung, die jedenfalls eine Aufnahme der Probe in einer Lösung hoher Salzkonzentration, vorzugsweise hoher Konzentration an chaotropen Ionen umfaßt, gewinnen. Auch Nucleinsäuren, die durch chemische Reaktionen entstanden sind, wie solche, die durch Polymerasekettenreaktion (PCR) erhalten wurden oder Plasmid-DNA, genomische DNA und RNA und/oder Nucleinsäuren, die aus Mikroorganismen stammen, lassen sich erfindungsgemäß trennen und reinigen.

Das erfindungsgemäße Verfahren ist ebenfalls geeignet, sogenannte Plasmid-DNA-Minipräparationen aus Escherichia Coli für die anschließende Klonierung oder Sequenzierung zu verwenden; ebenfalls geeignet ist das erfindungsgemäße Verfahren zur Isolierung von DNA und/oder RNA aus Vollblut, Plasma, Serum, Geweben, Zellkultur, Bakterien, insbesondere Mycobakterium tuberlosis, Viren wie Cytomegalie-Virus (Nucleinsäure DNA) aus RNA-Viren wie HIV, Hepatitis B, Hepatitis C, Hepatitis-δ. Nucleinsäuren im Sinne des erfindungsgemäßen sind auch Oligonucleotide. Die Nucleinsäuren können desweiteren aus Sequenzierreaktionen oder anderen vergleichbaren Reaktionen stammen. Die Präparation von DNA oder RNA aus Vollblut ist inbesondere geeignet zur anschließenden HLA-Typisierung. Das erfindungsgemäße Verfahren ist insbesondere geeignet zur Isolierung von Nucleinsäuren aus Mycobakterium tuberkulosis. Hierbei müssen recht drastische Aufschlußmethoden eingehalten werden, wobei konventionelle Isolierungstechniken nur unbefriedigende Resultate liefern.

Eine im erfindungsgemäßen Verfahren vorzugsweise zu verwendende Vorrichtung ist ein insbesondere zylindrischer Hohlkörper mit einer Ein- und Auslaßöffnung. In der Nähe der Auslaßöffnung, in Fließrichtung der Lösung durch den Hohlkörper gesehen, ist das mineralische Trägermaterial angeordnet, an welchem die Nucleinsäuren zu adsorbiert werden. Eine Einrichtung, die in einer bevorzugten Ausführungsform aus zwei übereinander angeordneten, einen Zwischenraum bildenden Polyethylenfritte besteht, fixiert das Trägermaterial, welches sich im Zwischenraum zwischen den Polyethylenfritten befindet, in dem Lumen des Hohlkörpers. Die Einrichtung zur Befestigung des Trägermaterials kann auch eine selbsttragende Membran sein, in der das Trägermaterial eingebettet ist. Die Befestigung des Trägermaterials bzw. der Einrichtungen, die das Trägermaterial fixieren, kann durch Reibungs- oder Spannungskräfte, wie sie beispielsweise durch Einklemmen dieser Einrichtungen in dem Hohlkörper entstehen, bewirkt werden und/oder durch eine Fixierung der Einrichtungen mit einem Spannring erfolgen.

Die Porengröße der Einrichtung, vorzugsweise Polyethylen - oder Polypropylenfritten, muß dabei groß genug sein, um die Bestandteile des Lysates verstopfungsfrei hindurchfließen zu lassen. Vorzugsweise haben die Einrichtungen eine Porengröße von 5 bis 200 *µ*m. Diese Vorrichtung erlaubt erstmals die einfache, schnelle und reproduzierbare Isolierung von Nucleinsäuren auch aus hochviskosen Lysaten, die sehr viel Protein enthalten (z.B. Blutlysate, welche einen sehr hohen Hämoglobingehalt aufweisen).

In einer besonders bevorzugten Ausführungsform ist das mineralische Trägermaterial eine netzartige Membran aus Silicagel-, Glas- oder Quarzfasern, mit einer Porengröße < 5 µm, an die die freigesetzten Nucleinsäuren adsorbiert werden.

Eine ebenfalls bevorzugte Ausführungsform stellt eine Vorrichtung dar, bei der das mineralische Trägermaterial ein partikelförmiges anorganisches Polymeres wie Kieselgel oder Quarzgel mit einer Partikelgröße von 1 bis 50 µm ist.

Der Hohlkörper kann zum Beispiel ein handelsübliches Röhrchen sein. Zwischen den zwei eng eingepreßten Einrichtungen, zum Beispiel Polyethylenfritten mit einer Porengröße von 50 bis 200 µm, befindet sich eine oder mehrere Lagen einer Membran mit 0,1 bis 1 µm großen Poren, welche aus Silica-, Glas- oder Quarzfasern oder Kieselgelen besteht. Die Membran weist eine Dicke von ca. 0,2 bis 1,0 mm, insbesondere 0,6 mm auf.

Die Kapazität des Membranmaterials ist etwa 20 bis 100 µg DNA. Durch Übereinanderlegen entsprechender Membranen läßt sich selbstverständlich die Kapazität für DNA erhöhen. Bei geringer mechanischer Belastung ist auch ein randständiges Verschweißen oder Verkleben der Membran denkbar, wodurch die stabilisierende Wirkung der Einrichtungen entfallen kann, so daß die Membran den Hohlkörper ohne die Einrichtungen verschließt. Dabei kann die Membran durch das Aufsetzen eines Spannringes im Hohlkörper fixiert werden.

Es ist ebenfalls möglich, mit dem beschriebenen Kieselgel, das zwischen 2 Polyethylen-Fritten mit einer Porengröße von 35 µm angeordnet ist, kleine Säulen zu füllen. Vorzugsweise wird die obere Einrichtung mit größeren Poren (10 - 250 µm, insbesondere 50 µm) gewählt. Die Säulen werden vorzugsweise mit ca. 70 mg Silicagel entsprechend 3 mm Füllhöhe beschickt.

Bevorzugt ist auch die Anwendung des oben beschriebenen Verfahrens in Strips mit je 8 parallelen Präparationsmöglichkeiten, im Mikrotitrationsplattenformat (96 Präparationsmöglichkeiten fast gleichzeitig) und/oder in Kombination mit einem Filtrationsschritt und/oder einem Entsalzungsschritt. (Siehe Patentanmeldungen P 41 27 276.5, P 41 39 664.2 des gleichen Anmelders).

In einer bevorzugten Ausführungsform der Vorrichtung wird eine 0,5 bis 1,5 mm dicke Polyethylenfritte mit einer Porosität von ca. 10 *µ*m in eine Zentrifugen-Chromatographiesäule in Form eines im wesentlichen zylindrischen Hohlkörpers eingeklemmt. Über diese Fritte ist eine etwa doppelt so dicke Schicht aus Silicagel einer Partikelgröße von etwa 10 bis 15 *µ*m und einer Porosität von 40 bis 120 Å und mit einer zweiten Fritte, die von gleicher Art sein kann wie die erste Fritte, verschlossen. Vorzugsweise kann die Silicagelschicht durch Druck zwischen den Fritten komprimiert werden.

Eine andere Ausführungsform der Chromatographiesäule weist als Trägermaterial Glasfaserbruchstücke mit einer Länge von 10 bis 300 µm zwischen zwei Polyethylenfritten auf, die eine Porosität von ca. 50 µm besitzen. Als Trägermaterial kommen auch Glasfaserpapiere, Quarzfaserpapiere, Glasfasergewebe und andere mineralische Papiere und Gewebe in Betracht.

Eine weitere bevorzugte Ausführungsform der Vorrichtung weist eine Membran, in die Silicagelpartikel eingebettet sind, in der Nähe der Auslaßöffnung auf. In diesem Fall kann die vorzugsweise selbsttragende Membran insbesondere mit einem Spannring fixiert werden. Als Silicagelmembran kommt in vorteilhafter Weise eine Empore-Silicagel-Membran der Firma 3M in Betracht. Ebenfalls, insbesondere mit einem Spannring, läßt sich eine Silicalgelmembran bestehend aus Silicagel und porösem PVC im Lumen des zylindrischen Hohlkörpers befestigen.

In einer bevorzugten Ausführungsform des erfindungsgemäßen Verfahrens wird die beschriebene Vorrichtung beispielsweise in einer ihrer Ausführungsformen mit der Lösung des zu trennenden Nucleinsäuregemisches beschickt. Die Lösung wird dann durch Anlegen eines Unterdrucks oder Zentrifugation oder gleichwirkende Maßnahme sowie Kombinationen davon durch den mineralischen Träger passiert. Dabei werden die Nucleinsäuren an dem Trägermaterial adsorbiert, sofern die Lösung eine hohe Ionenstärke (Salzkonzentration) aufweist.

Die Erfindung wird anhand der nachfolgenden Beispiele näher erläutert.

### Beispiel 1

### Isolierung von high copy Plasmid-DNA

Das Plasmid pUC 18 enthaltende E. coli Zellen aus einer 3 ml HB 101 Kultur werden abzentrifugiert und in 0,25 ml Puffer Pl (10 mM Tris-HCl, pH 8, 100 *µ*g/ml RNAseA) resuspendiert und durch die Zugabe von 0,25 ml Puffer P2 (0,2 M NaOH, 2% SDS) lysiert. Die Probe wird durch die Zugabe von 0,35 ml Puffer N3 (4,2 M Guanidinhydrochlorid, 0,9 M K-Acetat, pH 4,8) neutralisiert und gleichzeitig auf eine Endkonzentration von 1,75 M GuHCl eingestellt. Diese Konzentration gewährleistet eine Bindung ohne weitere Schritte. Die lysierte Probe wird 10 min. bei 13.000 rpm in einer Eppendorf-Minizentrifuge abzentrifugiert, um die Zellbruchstücke und das ausgefallene SDS zu entfernen. Der Überstand mit der Plasmid-DNA wird sofort auf eine Zentrifugen-Chromatographiesäule pipettiert. Die Zentrifugen-Chromatographiesäule wird in einem 2 ml Zentrifugenröhrchen zentrifugiert und durch nochmaliges Zentrifugieren von 0,5 ml PB Puffer (5 M Guanidinhydrochlorid, 30 % Isopropanol) gewaschen, um Verunreinigungen und Proteine zu entfernen. Die Zentrifugen-chromatographiesäule wird 1 x durch Durchzentrifugieren von 80 % Ethanol/Wasser salzfrei gewaschen und anschließend 30 bis 60 sec. zentrifugiert, um das überschüssige Ethanol komplett zu entfernen. Zur Elution werden 0,05 - 0,2 ml Elutionspuffer (10 mM Tris-HCl, pH 8,5) durch die Zentrifugen-Chromatographiesäule in ein 1,5 ml- Zentrifugenröhrchen zentrifugiert. Die Plasmid-DNA liegt jetzt in konzentrierter Form in einer Lösung mit sehr niedriger Salzkonzentration vor. Die Ausbeute beträgt 15 µg bis 20 µg Plasmid-DNA mit einem A260/A280 Verhältnis von 1,75.

### Beispiel 2

### Isolierung von high copy Plasmid-DNA aus 5 ml Kulturen

Nach Beispiel 1 wird ein Zell-Lysat einer 5 ml Plasmid pUC 18/XL 1 Blue Kultur hergestellt und durch eine Zentrifugen-Chromatographiesäule, welche eine Silicalgelschüttung enthält, zentrifugiert und gewaschen. Die Plasmid-DNA wird mit 0,1 ml auf 80°C erwärmtem TE-Puffer (10 mM Tris-HCl, pH 8,5, 1 mM EDTA) eluiert. Die Ausbeute beträgt 15 - 20 µg Plasmid DNA mit einem A260/A280 Verhältnis von 1,7.

### Beispiel 3

### Isolierung von low copy Plasmid-DNA

Nach Beispiel 1 wird ein Zell-Lysat einer 5 ml Plasmid pBR322/XL 1 Blue Kultur hergestellt und durch eine Zentrifugen-Chromatographiesäule mit einer Glas- oder Quarzfasermembran zentrifugiert und gewaschen. Die Plasmid-DNA wird mit 0,1 ml auf 80°C erwärmten TE-Puffer (10 mM Tris-HCl, pH 8,5, 1mM EDTA) eluiert. Die Ausbeute beträgt 5 - 10 µg Plasmid DNA mit einem A260/A280 Verhältnis von 1,7.

### Beispiel 4

### Reinigung von Amplifikationsprodukten

Eine 100 µl PCR-Amplifikationsreaktion wird mit 500 µl Puffer PB (5 M GuHCl, 30% Isopropanol) vermischt, wobei ein vorheriges Abtrennen des den Reaktionsansatz überschichtenden Paraffinöls nicht nötig ist. Dieses Gemisch wird auf eine Zentrifugen-Chromatographiesäule, welche eine Silicagelmembran enthält, pipettiert und in einem 1,5 ml Zentrifugenröhrchen zentrifugiert. Die Zentrifugen-Chromatographiesäule wird durch Behandeln mit 80% EtOH/Wasser annähernd salzfrei gewaschen. Zur Elution werden 50 µl Elutionspuffer (10 mM Tris, pH 8,5) durch die Zentrifugen-Chromatographiesäule in ein anderes Zentrifugenröhrchen zentrifugiert. Das so gereinigte PCR Produkt ist frei von Primern, dNTPs, Polymerase und Salzen und kann beispielsweise direkt für eine Sequenzierungsreaktion in einem ABI-Sequencer unter Verwendung des "Cyle-Sequencing" Protokolls eingesetzt werden.

### Beispiel 5

### Reinigung von DNA nach Restriktionsreaktionen

1 µg DNA wird mit einer Restriktionsendonuclease behandelt. Diese DNA Restriktionsreaktion wird nach Beispiel 4 mit 500 µl PB Puffer vermischt und es wird weiter wie in Beispiel 4 verfahren. Die nach Elution erhaltene DNA ist frei von Restriktionsendonucleasen und Salzen, das 260/280 Verhältnis beträgt 1,8.

### Beispiel 6

### Reinigung von DNA nach enzymatischer radioaktiver Markierung

1 µg DNA werden mit Hilfe des Oligolabellings, in Anwesenheit von Gamma P32-ATP radioaktiv markiert. Der Reaktionsansatz wird wie in Beispiel 4 behandelt. Hierdurch wird die markierte DNA von nicht eingebauten dNTPs, Gamma-P32 ATP, Salzen und Klenow-Polymerase gereinigt und kann direkt für die Hybridisierungsreaktion eingesetzt werden.

### Beispiel 7

### Nucleinsäure-Präparation aus Blut

Gesamt-Nucleinsäure-Präparation aus Blut: 200 µl Citrat-, Heparin- oder EDTA-Blut werden in einem 1.5 ml PPN-Röhrchen mit 200 µl einer Lösung aus einem 4 - 8 M chaotropen Salz (Guanidinhydrochlorid (GuHCl), Guanidinisothiocyanat (GTC), Kaliumjodid), evtl. einem organischen Lösungsmittel (Phenol, Chloroform, Ether) und einem 5 - 100 %igen Detergens (NP4O; Tween 20, Triton X-100, SDS, CTAB) versetzt. Anschließend werden 200 -1000 µg einer Protease zugefügt und es wird für 10 min. bei 70°C oder für längere Zeit bei niedrigeren Temperaruren (z.B. 30 min bei Raumtemperatur) inkubiert. In diesem Schritt erfolgen gleichzeitig die effiziente Lyse aller eukaryontischen und/oder prokaryontischen Zellen und/oder Viren (gleichzeitige Inaktivierung infektiöser Pathogene) und Denaturierung und enzymatischer Abbau von Proteinen (gleichzeitig Entfernung der an die Nucleinsäuren gebundenen Proteine). Durch Zugabe von 210 µl eines 95 - 100 %igen Alkohols (Methanol, Ethanol, n-Propanol, Isopropanol, PEG, sekundäre und tertiäre, kurz- oder langkettige Alkohole) werden für Nucleinsäuren hochspezifische Bindebedingungen erzeugt und das so eingestellte Lysat wird auf die Vorrichtung aufgetragen. Durch Zentrifugation oder Druck wird das Lysat sodann durch die Membran oder Gelmatrix geleitet, wobei die Nucleinsäuren reversibel an die Membranfasern oder Gelpartikel binden. Mit 0,7 ml 100 mM NaCl, 10 mM Tris-HCl pH 7,5, 30 - 80% eines reinen Alkohols (Methanol, Ethanol, n-Propanol, Isopropanol, PEG, sekundäre und tertiäre, kurz- oder langkettige Alkohole) oder eines Alkoholgemisches werden die Verunreinigungen wie Proteine, Häm, Heparin, Eisenionen, Metabolite etc. ausgewaschen. Die DNA wird entweder mit einem Niedrigsalzpuffer (10 mM Tris-HCl pH 9,0) oder destiliertem (deionisierten) Wasser eluiert. Der Vorteil dieser Elutionsverfahren besteht darin, daß die so gewonnene DNA direkt ohne weitere Fällungs- oder Umpufferungsschritte in Folgereaktionen, insbesondere der PCR, eingesetzt werden können. Die Präparation von Nucleinsäuren aus anderen Körperflüssigkeiten wie z.B. Sperma, Sputum, Urin, Stuhl, Schweiß, Speichel, Nasenschleim, Serum, Plasma, Liquor etc. ist auch möglich.

Diese einfache Methode zur Isolierung von Nucleinsäuren weist ein hohes Automatisierungspotential insbesondere in Verbindung mit den Anmeldungsgegenständen der DE-A 41 27 276.5, WO 93/11218, WO 93/11211 und DE-A 41 39 664.2 auf.

### Beispiel 8

### Gesamt-Nucleinsäure-Präparation aus geringsten Blutmengen oder Spuren

1 - 50 µl Citrat-, Heparin- oder EDTA-Blut, oder gefrorenes und wieder aufgetautes Blut, oder renaturiertes Blut aus getrockneten Spuren in Textilgewebe, werden in einem 1,5 ml PPN-Röhrchen mit 1 - 50 µl einer Lösung aus einem 4 - 8 M chaotropen Salz (Guanidinhydrochlorid, Guanidinisothiocyanat, Kaliumjodid), evtl. einem organischen Lösungsmittel (Phenol, Chloroform, Ether) und einem 1 - 100 %igen Detergenz (NP4O; Tween 20, Triton X-100, SDS, CTAB) versetzt. Anschließend werden 1 - 200 µg einer Protease zugefügt und es wird für 1 min. bei 70°C oder für längere Zeit bei niedrigeren Temperaturen (z.B. 10 min bei Raumtemperatur) inkubiert.

In diesem Schritt erfolgen gleichzeitig die effiziente Lyse aller eukaryontischen und/oder prokaryontischen Zellen und/oder Viren (gleichzeitige Inaktivierung infektiöser Pathogene) und Denaturierung und enzymatischer Abbau von Proteinen (gleichzeitig Entfernung der an die Nucleinsäuren gebundenen Proteine). Durch Zugabe von 1/2 Volumen eines 95 - 100 %igen Alkohols (Methanol, Ethanol, n-Propanol, Isopropanol, sekundäre und tertiäre, kurz- oder langkettige Alkohole) oder organischen Polymeren (PEG) werden für Nucleinsäuren hochspezifische Bindebedingungen erzeugt und das so eingestellte Lysat wird auf die Vorrichtung aufgetragen. Durch Zentrifugation oder Druck wird das Lysat sodann durch die Membran oder Gelmatrix geleitet, wobei die Nucleinsäuren reversibel an die Membranfasern oder Gelpartikel binden. Mit 0,7 ml 100 mM NaCl, 10 mM Tris-HCl pH 7,5, 30 - 80% eines reinen Alkohols (Methanol, Ethanol, n-Propanol, Isopropanol, PEG, sekundäre und tertiäre, kurz- oder langkettige Alkohole) oder eines Alkoholgemisches werden die Verunreinigungen wie Proteine, Häm, Heparin, Eisenionen, Metabolite etc. ausgewaschen. Die DNA wird entweder mit einem Niedrigsalzpuffer (10 mM Tris-HCl pH 9,0) oder destilliertem (deionisierten) Wasser eluiert. Der Vorteil dieser Elutionsverfahren besteht darin, daß die so gewonnene DNA direkt ohne weitere Fällungs- oder Umpufferungsschritte in Folgereaktionen, insbesondere der PCR, eingesetzt werden kann.

Dieses Verfahren ist auch zur Präparation von Nucleinsäuren aus kleinsten Mengen anderer Körperflüssigkeiten (Sperma, Sputum, Urin, Stuhl, Schweiß, Speichel, Nasenschleim, Serum, Plasma, Liquor etc.) oder getrockneten Spuren derselben geeignet.

Diese einfache Methode zur Isolierung von Nucleinsäuren weist ein hohes Automatisierungspotential insbesondere in Verbindung mit den Anmeldungsgegenständen der DE-A 41 27 276.5, WO 93/11218, WO 93/11211 und DE-A 41 39 664.2 auf.

### Beispiel 9

### Gesamt-Nucleinsäure-Präparation aus Gewebe

100 µg bis 10 mg eines Gewebes werden in einem PPN-Röhrchen mit einer Lösung aus einem 4 - 8 M chaotropen Salz (Guanidinhydrochlorid, Guanidinisothiocyanat, Kaliumjodid), evtl. einem organischen Lösungsmittel (Phenol, Chloroform, Ether) und einem 5 - 100 %igen Detergens (NP4O; Tween 20, Triton X-100, SDS, CTAB) versetzt und mit Hilfe eines kommerziell erhältlichen Homogenisators oder durch Mörsern in flüssigem Stickstoff homogenisiert. Anschließend werden 100 - 1000 µg einer Protease zugefügt und es wird für 10 - 20 min. bei 70°C oder für längere Zeit bei niedrigeren Temperaruren (z.B. 30 - 60 min bei Raumtemperatur) inkubiert. In diesem Schritt erfolgen gleichzeitig die effiziente Lyse aller eukaryontischen und/oder prokaryontischen Zellen und/oder Viren (gleichzeitige Inaktivierung infektiöser Pathogene) und Denaturierung und enzymatischer Abbau von Proteinen (gleichzeitig Entfernung der an die Nucleinsäuren gebundenen Proteine). Durch Zugabe von % Volumen eines 95 - 100 %igen Alkohols (Methanol, Ethanol, n-Propanol, Isopropanol, PEG, sekundäre und tertiäre, kurz- oder langkettige Alkohole) werden für Nucleinsäuren hochspezifische Bindebedingungen erzeugt und das so eingestellte Lysat wird auf die Vorrichtung aufgetragen. Durch Zentrifugation oder Druck wird das Lysat sodann durch die Membran oder Gelmatrix geleitet, wobei die Nucleinsäuren reversibel an die Membranfasern oder Gelpartikel binden. Mit 0,7 ml 100 mM NaCI, 10 mM Tris-HCl pH 7,5, 30 - 80% eines reinen Alkohols (Methanol, Ethanol, n-Propanol, Isopropanol, PEG, sekundäre und tertiäre, kurz-oder langkettige Alkohole) oder eines Alkoholgemisches werden die Verunreinigungen wie Proteine, Häm, Heparin, Eisenionen, Metabolite etc. ausgewaschen. Die DNA wird entweder mit einem Niedrigsalzpuffer (10 mM Tris-HCl pH 9,0) oder destilliertem (deionisierten) Wasser eluiert. Der Vorteil dieser Elutionsverfahren besteht darin, daß die so gewonnene DNA direkt ohne weitere Fällungs- oder Umpufferungsschritte in Folgereaktionen, insbesondere der PCR, eingesetzt werden können.

Diese einfache Methode zur Isolierung von Nucleinsäuren funktioniert reproduzierbar aus allen Geweben, u.a. auch aus Tumoren und weist ein hohes Automatisierungspotential, insbesondere in Verbindung mit den Anmeldungsgegenständen der DE-A-41 27 276.5, WO 93/11218, Wo 93/11211 und DE-A-41 39 664.2 des gleichen Anmelders auf.

### Beispiel 10

### Reinigung von DNA-Fragmenten aus Agarosegelen

Ein DNA-Fragment wird in einem Agarosegel (TAE oder TBE 0,5 - 2% aufgetrennt. Das zu isolierende DNA-Fragment wird aus dem Gel ausgeschnitten und in einem 1,5 ml Eppendorf-Gefäß mit 300 µl QX1 Puffer (7 M NaPO₄, 10 mM NaAc, pH 5,3) vermischt. Nach 10 minütiger Inkubation bei 50°C hat sich die Agarose aufgelöst. Diese Lösung wird auf eine Zentrifugen-Chromatographiesäule nach Beispiel 1 gegeben und durchzentrifugiert. Die Zentrifugen-Chromatographiesäule wird nun mittels Zentrifugieren von 80% Ethanol/Wasser durch die Säule salzfrei gewaschen und anschließend 1 min. zentrifugiert, um überschüssiges Ethanol komplett zu entfernen. Zur Elution werden 0,05 ml Elutionspuffer (10 mM Tris-HCl, pH 8,5) auf die Chromatographiesäule gegeben und durchzentrifugiert.

### Beispiel 11

### Reinigung von DNA-Fragmenten aus Polyacrylamid (PAA)-Gelen

Das zu isolierende DNA-Fragment wird aus dem PAA-Gel ausgeschnitten und in ein 2 ml Eppendorf-Gefäß überführt, zerkleinert und mit 500 µl PAA-Elutionspuffer (500 mM NH₄Ac, 100 mM MgAc₂, 1 mM EDTA, 0,1 % SDS) vermischt. Das Gemisch wird 30 min. bei 50°C inkubiert und anschließend mit 300 µl QX1 Puffer versetzt und über eine Zentrifugen-Chromatographiesäule zentrifugiert. Die Zentrifugen-Chromatographiesäule wird nun mit 80% Ethanol/Wasser salzfrei gewaschen und anschließend 1 min. zentrifugiert, um überschüssiges Ethanol komplett zu entfernen. Zur Elution werden 0,1 ml Elutionspuffer (10 mM Tris-HCl, pH 8,5) auf die Chromatographiesäule gegeben und durchzentrifugiert.

### Beispiel 12

### Reinigung von großen (> 3.000 bp) PCR-Fragementen

Eine 100 µl PCR-Amplifikationsreaktion wird mit 500 µl Puffer QXB (5 M GuHCl) vermischt, wobei ein vorheriges Abtrennen des den Reaktionsansatz überschichtenden Paraffinöls nicht nötig ist. Zur weiteren Aufreinigung wird wie in Beispiel 4 verfahren.

### Beispiel 13

### Reinigung von einzelsträngigen PCR-Produkten

Ein 100 µl Amplifikationsansatz einer asymetrischen PCR wird mit 500 µl PB-Puffer (5 M GuHC, 30 % Isopropanol) vermischt. Zur weiteren Aufreinigung wird wie in Beispiel 4 verfahren. Zur Elution werden 0,05 ml Elutionspuffer (10 mM Tris-HCl, pH 8,5) auf die Chromatographiesäule gegeben und durchzentrifugiert. Das Eluat enthält ca. 90% des einzelsträngigen PCR-Produktes, welches direkt für die zweite Amplifikation oder zum Sequenzieren eingesetzt werden kann.

### Beispiel 14

### Gesamt-Nucleinsäurepräparation aus Gewebe oder Zellen

Bis zu 50 mg eines Gewebes oder bis zu 10⁶ Zellen werden in 400 µl einer gepufferten chaotropen Lösung (4 M GTC, 25 mM Natriumcitrat pH 7,5, 2% 2-Mercaptoethanol) - gegebenenfalls versetzt mit einem 5 - 100 %-igem Detergens (NP40, Tween 20, Triton-X-100, SDS, CTAB, Sarkosyl) - homogenisiert. In diesem Schritt erfolgen gleichzeitig die effiziente Lyse aller eukaryontischer und/oder prokaryontischen Zellen und/oder Viren (gleichzeitige Inaktivierung infektiöser Pathogene) und Denaturierung von Proteinen (insbesondere Ribonucleasen; gleichzeitig Entfernung der an die Nucleinsäuren gebundenen Proteine). Durch Zugabe von 260 µl eines 100 %-igen Alkohols (Methanol, Ethanol, n-Propanol, Isopropanol) werden für Nucleinsäuren hochspezifische Bindebedingungen erzeugt. Das so eingestellte Lysat wird auf die Vorrichtung aufgetragen.

Anschließend werden Verunreinigungen wie Proteine, Häm, Heparin, Metabolite und Polysaccharide mit 700 µl eines Waschpuffers bestehend aus 1 M GTC, 25 mM Tris/HCl, pH 7,5, 40 % eines Alkoholes (Methanol, Ethanol, n-Propanol, Isopropanol) sowie 700 µl eines Waschpuffers bestehend aus 10 mM Tris/HCl, pH 7,5, 80% eines Alkoholes (Methanol, Ethanol, n-Propanol, Isopropanol) ausgewaschen. Die Nucleinsäuren werden entweder mit einem Niedrigsalzpuffer (10 mM Tris, pH 7,5) oder destilliertem (deionisiertem) Wasser eluiert.

## Patentansprüche

1. Verfahren zur chromatographischen Reinigung und Trennung von Nucleinsäuregemischen, wobei das Nukleinsäuregemisch
• aus einer wässrigen Adsorptionslösung mit hoher Salzkonzentration (Ionenstärke) und mit 1 bis 50 Vol.-% aliphatischen Alkohols einer Kettenlänge von C₁-C₅ und/oder Polyethylenglykol (PEG) und/oder hydrophober, anorganischen und/oder organischen Polymeren und/oder organischer Säure, wie Trichloressigsäure (TCA),
• an einem porösen oder nicht porösen mineralischen Träger aus Metalloxiden und/oder Metallmischoxiden, Silicalgel, Materialien, die überwiegend aus Glas bestehen, Aluminiumoxid, Zeolithe, Titandioxid, Zirkondioxid - ohne vorherige Reinigungsschritte - adsorbiert wird,
• gegebenenfalls mit einer Waschlösung gewaschen und danach
• mit einer Lösung geringerer Salzkonzentration (Ionenstärke) eluiert wird und die erhaltene Nucleinsäuren oder Nucleinsäurefraktionen gesammelt werden.

2. Verfahren nach Anspruch 1, wobei als Salze In der Adsorptionslösung chaotrope Salze verwendet werden wie Natriumperchlorat, Guanidinhydrochlorid, Guanidinisothiocyanat, Natriumjodid in Konzentrationen von 1 bis 8 M.

3. Verfahren nach Anspruch 1 und/oder 2, wobei die Ionenstärken der Salzlösungen mit 1 bis 10 M Lithiumchlorid, Natriumchlorid, Kaliumchlorid, Natriumacetat, Reagenzien wie z.B. Harnstoff und/oder Mischungen davon eingestellt wird.

4. Verfahren nach mindestens einem der Ansprüche 1 bis 3, wobei die Partikelgröße des mineralischen Trägermaterials 0,1 µm bis 1.000 µm beträgt.

5. Verfahren nach mindestens einem der Ansprüche 1 bis 4, wobei verwendete poröse mineralische Trägermaterialien eine Porengröße von 2 bis 1.000 nm aufweisen.

6. Verfahren nach mindestens einem der Ansprüche 1 bis 5, wobei die porösen oder nicht porösen Trägermaterialien, insbesondere Zeolithen, in Form von losen Schüttungen vorliegen.

7. Verfahren nach mindestens einem der Ansprüche 1 bis 5, wobei die porösen oder nicht porösen Trägermaterialien, insbesondere Zeolithen, als Filterschichten ausgebildet sind in Form von Filterschichten aus Glas, Quarz oder Keramik und/oder einer Membran, in der Silicagel angeordnet ist und/oder als Partikel oder Fasern aus mineralischen Trägem und Geweben aus Quarz oder Glaswolle vorliegen.

8. Verfahren nach mindestens einem der Ansprüche 1 bis 7, wobei die zu trennenden und zu reinigenden Nucleinsäuren aus biologischen Quellen wie Zellkulturen, Geweben jeder Art, Körperflüssigkeiten wie Blut, Plasma, Serum, Urin, Faeces, Mikroorganismen wie Bakterien, Insbesondere Mycobakteriumtuberkulosis, Viren wie Cytomegalie-Virus, HIV, Hepatitis B, Hepatitis C, Hepatitis-δ-Virus, stammen, die Nucleinsäuren durch Polymerasekettenreaktion (PCR) erhaltene Produkte, Plasmid-DNA, genomische DNA, RNA, Nucleinsäuren aus Mikroorganismen wie Plasmid-DNA, chromosomale DNA oder RNA und/oder Nucleinsäuren aus Sequenzanalysen sind.

9. Verfahren nach mindestens einem der Ansprüche 1 bis 8, wobei die Nucleinsäuren nach Fraktionierung zu weniger als 10% kürzer als 10 kb sind.

10. Verfahren nach mindestens einem der Ansprüche 1 bis 9, wobei die Nucleinsäuren Oligonucleotide sind.

11. Verfahren nach mindestens einem der Ansprüche 1 bis 10, wobei nach Lyse einer nukleinsäurehaltigen Probe als Quelle der zu reinigenden und isolierenden Nucleinsäure die Einstellung von Adsorptionsbedingungen an Silicagel in einem einzigen Schritt erfolgt, wobei die Adsorptionsbedingungen durch Lyse der Proben in dem Puffersystem, das zur Adsorption eingesetzt wird, eingestellt werden.

12. Verfahren nach Anspruch 11, wobei zur Präparation von Plasmiden nach der alkalischen Lyse der Probe die Einstellung von Adsorptionsbedingungen an Silicagel in einem einzigen Schritt erfolgt.

13. Verfahren nach einem der Ansprüche 1 bis 12, wobei zur Plasmidpräparation die Adsorption des Plasmids in Gegenwart von Detergenzien erfolgt.

14. Verwendung des Verfahrens nach mindestens einem der Ansprüche 1 bis 13 zur Reinigung und Trennung von Nucleinsäurefragmenten nach Modifizierungsreaktionen.

15. Verwendung einer Vorrichtung aus einem Hohlkörper mit Ein- und Auslassöffnung, wobei im Lumen des Hohlkörpers eine Einrichtung angeordnet ist, die das Trägermaterial zur Adsorption der zu trennenden Nudeinsäuren fixiert, dadurch gekennzeichnet, dass
• die Einrichtung zwei übereinander angeordnete, einen Zwischenraum ausbildenden Polyethlenfritten sind, wobei das Trägermaterial zwischen den Polyethylenfritten angeordnet ist oder
• die Einrichtung eine selbsttragende Membran ist, in welcher das Trägermaterial eingebettet ist.
• zur Durchführung des Verfahrens nach einem der Ansprüche 1 bis 13.

16. Verwendung nach Anspruch 15, wobei die Befestigung der Einrichtungen im Lumen des Hohlkörpers durch Reibungs- und/oder Spannungskräfte und/oder durch Fixierung mit einem Spannring erfolgt.

## Claims

1. A process for the purification and separation of nucleic acid mixtures by chromatography, wherein said nucleic acid mixture
- is adsorbed, without any previous purification step, from an aqueous adsorption solution having a high salt concentration (ionic strength) and comprising from 1 to 50% by volume of aliphatic alcohol having a chain length of C₁-C₅ and/or polyethylene glycol (PEG) and/or hydrophobic inorganic and/or organic polymers and/or organic acid, such as trichloroacetic acid (TCA);
- to a porous or non-porous mineral support of metal oxides and/or metal mixed oxides, silica gel, alumina, zeolites, titania, zirconia, materials predominantly consisting of glass;
- is optionally washed with a washing solution; and thereafter
- is eluted with a solution having a lower salt concentration (ionic strength), and the nucleic acids or nucleic acid fractions obtained are collected.

2. The process according to claim 1, wherein chaotropic salts such as sodium perchlorate, guanidine hydrochloride, guanidinium isothiocyanate or sodium iodide in concentrations of from 1 to 8 M are used as the salts in said adsorption solution.

3. The process according to claim 1 and/or 2, wherein the ionic strengths of the salt solutions are adjusted with from 1 to 10 M lithium chloride, sodium chloride, potassium chloride, sodium acetate, reagents such as urea, and/or mixtures thereof.

4. The process according to at least one of claims 1 to 3, wherein the particle size of the mineral support material is from 0.1 µm to 1,000 µm.

5. The process according to at least one of claims 1 to 4, wherein porous mineral support materials, when employed, have a pore size of from 2 to 1,000 nm.

6. The process according to at least one of claims 1 to 5, wherein the porous or non-porous support materials, especially zeolites, are in the form of loose packings.

7. The process according to at least one of claims 1 to 5, wherein the porous or non-porous support materials, especially zeolites, are designed as filter sheets in the form of glass, quartz or ceramic filter sheets, and/or a membrane containing silica gel, and/or as particles or fibers of mineral supports and fabrics of quartz or glass wool.

8. The process according to at least one of claims 1 to 7, wherein the nucleic acids to be separated and purified are derived from biological sources, such as cell cultures; all kinds of tissues; body fluids, such as blood, plasma, serum, urine, faeces; microorganisms, such as bacteria, especially Mycobacterium tuberculosis, viruses, such as cytomegalovirus, HIV, hepatitis B, hepatitis C, hepatitis δ viruses; and the nucleic acids are products obtained by polymerase chain reaction (PCR), plasmid DNA, genomic DNA, RNA, nucleic acids from microorganisms, such as plasmid DNA, chromosomal DNA or RNA, and/or nucleic acids from sequence analyses.

9. The process according to at least one of claims 1 to 8, wherein, after fractionating, less than 10% of the nucleic acids is molecules which are shorter than 10 kb.

10. The process according to at least one of claims 1 to 9, wherein said nucleic acids are oligonucleotides.

11. The process according to at least one of claims 1 to 10, wherein lysis of a sample containing nucleic acids as a source of the nucleic acid to be purified and isolated is followed by adjusting adsorption conditions in silica gel, in a single step, the adsorption conditions being adjusted by lysis of the samples in the buffer system employed for adsorption.

12. The process according to claim 11, wherein alkaline lysis of the sample is followed by adjusting adsorption conditions in silica gel, in a single step, for the preparation of plasmids.

13. The process according to any of claims 1 to 12, wherein the adsorption of a plasmid, for the preparation of plasmids, is effected in the presence of detergents.

14. Use of the process according to at least one of claims 1 to 13 for the purification and separation of nucleic acid fragments following modification reactions.

15. Use of a device consisting of a hollow body having an inlet and an outlet, wherein a means for fixing the support material for adsorption of the nucleic acids to be separated is provided in the lumen of said hollow body, characterized in that
- said means is two polyethylene frits arranged at a vertical distance leaving a space therebetween, the support material being positioned between said polyethylene frits; or
- said means is an integral membrane in which the support material is embedded;
- for performing the process according to any of claims 1 to 13.

16. The use according to claim 15, wherein the attachment of said means within the lumen of said hollow body is effected by frictional and/or tensional forces and/or by fixing with a locking ring.

## Revendications

1. Procédé de purification et de séparation chromatographique de mélanges d'acides nucléiques, dans lequel le mélange d'acides nucléiques
• est adsorbé, à partir d'une solution aqueuse d'adsorption à forte concentration de sels (force ionique) et contenant de 1 à 50 % en volume d'un alcool aliphatique ayant une longueur de chaîne en C₁-C₅ et/ou de polyéthylèneglycol (PEG) et/ou de polymères hydrophobes, inorganiques et/ou organiques, et/ou d'un acide organique tel que l'acide trichloracétique (TCA),
• par un support minéral, poreux ou non-poreux, en oxydes métalliques et/ou oxydes mixtes métalliques, gel de silice, en des matériaux qui pour l'essentiel sont constitués de verre, en oxyde d'aluminium, zéolites, dioxyde de titane, dioxyde de zirconium - sans opération préalable d'épuration -,
• est éventuellement lavé avec une solution de lavage, puis
• est élué avec une solution à faible concentration de sels (force ionique), et les acides nucléiques ou fractions d'acides nucléiques obtenus sont recueillis.

2. Procédé selon la revendication 1, dans lequel on utilise en tant que sels dans la solution d'adsorption des sels chaotropes tels que le perchlorate de sodium, le chlorhydrate de guanidine, l'isothiocyanate de guanidine, l'iodure de sodium, à des concentrations de 1 à 8 M.

3. Procédé selon la revendication 1 et/ou 2, dans lequel la force ionique des solutions de sels est ajustée avec 1 à 10 M de chlorure de lithium, de chlorure de sodium, de chlorure de potassium, d'acétate de sodium, de réactifs tels par exemple que l'urée, et/ou des mélanges de ceux-ci.

4. Procédé selon au moins l'une des revendications 1 à 3, dans lequel la granulométrie du matériau support minéral est de 0,1 µm à 1000 µm.

5. Procédé selon au moins l'une des revendications 1 à 4, dans lequel les matériaux supports minéraux poreux utilisés ont un diamètre de pores de 2 à 1000 nm.

6. Procédé selon au moins l'une des revendications 1 à 5, dans lequel les matériaux supports poreux ou non-poreux, en particulier les zéolites, se présentent sous forme de matériaux meubles en vrac.

7. Procédé selon au moins l'une des revendications 1 à 5, dans lequel les matériaux supports poreux ou non-poreux, en particulier les zéolites, sont conformés comme des couches filtrantes, sous forme de couches filtrantes en verre, quartz ou céramique, et/ou sous forme d'une membrane dans laquelle est disposé un gel de silice, et/ou se présentent sous forme de particules ou de fibres constituées de supports minéraux et de tissus de quartz ou de laine de verre.

8. Procédé selon au moins l'une des revendications 1 à 7, dans lequel les acides nucléiques à séparer et à purifier proviennent de sources biologiques telles que les cultures cellulaires, les tissus de tous types, les liquides corporels tels que le sang, le plasma, le sérum, l'urine, les fèces, les microorganismes tels que les bactéries, en particulier Mycobacterium tuberculosis, les virus tels que les cytomégalovirus, le VIH, le virus de l'hépatite B, le virus de l'hépatite C, le virus de l'hépatite δ, les acides nucléiques étant des produits obtenus par amplification en chaîne par polymérase (PCR), les ADN plasmidiques, les ADN génomiques, les ARN, les acides nucléiques provenant de microorganismes tels que les ADN plasmidiques, les ADN chromosomiques ou les ARN, et/ou les acides nucléiques provenant d'analyses de séquences.

9. Procédé selon au moins l'une des revendications 1 à 8, dans lequel, après fractionnement, moins de 10 % des acides nucléiques ont une taille inférieure à 10 kb.

10. Procédé selon au moins l'une des revendications 1 à 9, dans lequel les acides nucléiques sont des oligonucléotides.

11. Procédé selon au moins l'une des revendications 1 à 10, dans lequel, après lyse d'un échantillon contenant un acide nucléique, servant de source de l'acide nucléique à purifier et à isoler, on procède à l'ajustement des conditions d'adsorption sur gel de silice dans une étape unique, les conditions d'adsorption étant ajustées par une lyse des échantillons dans le système tampon utilisé pour l'adsorption.

12. Procédé selon la revendication 11, dans lequel, pour préparer les plasmides après la lyse alcaline de l'échantillon, on procède à l'ajustement des conditions d'adsorption sur gel de silice en une étape unique.

13. Procédé selon l'une des revendications 1 à 12, dans lequel, pour préparer le plasmide, on procède à l'adsorption du plasmide en présence de détergents.

14. Utilisation du procédé selon au moins l'une des revendications 1 à 13 pour purifier et séparer des fragments d'acides nucléiques après des réactions de modification.

15. Utilisation d'un équipement constitué d'un corps creux, avec un orifice d'entrée et un orifice de sortie, un dispositif qui fixe le matériau support pour adsorption des acides nucléiques à séparer étant disposé dans la lumière du corps creux, caractérisée en ce que
• le dispositif est constitué de deux frittes de polyéthylène disposées l'une au-dessus de l'autre, formant un espace intermédiaire, le matériau support étant disposé entre les frittes de polyéthylène, ou
• le dispositif est une membrane autoportante dans laquelle est incorporé le matériau support,
• pour mettre en oeuvre le procédé selon l'une des revendications 1 à 13.

16. Utilisation selon la revendication 15, dans laquelle la fixation des dispositifs dans la lumière du corps creux est réalisée par des forces de frottement et/ou de tension et/ou par fixation à l'aide d'une bague de serrage.
